# EUROPEAN PATENT APPLICATION

(11) **EP 4 574 075 A1**
(43) Date of publication of application: **25.06.2025**
(21) Application number: 24221349.4
(22) Date of filing: 19.12.2024
(51) Int. Cl.: A61B 18/14, A61B 18/00, A61B 90/00

(54) **IRRIGATED ELECTROPHYSIOLOGY BALLOON CATHETER**

(30) Priority: 20.12.2023 US 202363612709 P; 25.11.2024 US 202418959187
(71) Applicant: Biosense Webster (Israel) Ltd., 2066717 Yokneam (IL)
(72) Inventor: RAMPA, Sreekanth Reddy, Irvine, 92618 (US); RODRIGUEZ, Jasson, Irvine, 92618 (US); GOVEA, Michael, Irvine, 92618 (US)
(74) Representative: Carpmaels & Ransford LLP

(57) **Abstract**

An electrophysiology catheter includes a balloon that may be expanded with an irrigation liquid, and that can have the irrigation liquid removed more quickly therefrom than other catheter balloons having a similar volume and without damaging electrical componentry disposed on the balloon. The catheter includes a shaft assembly comprising a shaft-assembly lumen, a choke lumen, and ports that connect the shaft-assembly lumen to an interior of the balloon. A guidewire may be disposed in the choke lumen to divert irrigation liquid into the balloon via the ports, which expands the balloon. Upon removal of the guidewire from the choke lumen, irrigation liquid can exit the balloon through the ports and then exit the catheter via its distal tip.

## Description

### CROSS-REFERENCE TO RELATED APPLICATIONS

This application claims priority under 35 U.S.C. § 119 to U.S. Provisional Patent Application No. 63/612,709, filed December 20, 2023. This application also relates to subject matter described in U.S. Patent Application No. 15/360,966, filed November 23, 2016, issued as U.S. Patent No. 10,660,700. The entire contents of these applications are incorporated by reference herein in their entirety.

### FIELD

The subject matter disclosed herein relates to ablation systems, particularly those that include a catheter capable of ablating cardiac tissue.

### BACKGROUND

Ablation of cardiac tissue may be used to treat cardiac arrhythmias. Ablative energies may be provided to cardiac tissue by structures, such as electrodes, disposed on a distal portion of a catheter. Some catheters have their electrodes disposed on or incorporated into three-dimensional structures, e.g., wire baskets and balloons.

### SUMMARY OF THE DISCLOSURE

An electrophysiology catheter includes a balloon that may be expanded with an irrigation liquid, and that can have the irrigation liquid removed more quickly therefrom than other catheter balloons having a similar volume and without damaging electrical componentry disposed on the balloon. The electrophysiology catheter includes a shaft assembly comprising a plurality of tubular components having respective walls that together define an interior of the shaft assembly that includes a shaft-assembly lumen. A first of the plurality of tubular components comprises a first shaft including a first-shaft wall that forms a first one of the respective walls, a first-shaft proximal portion, a first-shaft distal portion, and a first-shaft lumen extending through the first shaft such that the first-shaft lumen forms a first portion of the interior of the shaft assembly. A second of the plurality of tubular components comprises a second shaft including a second-shaft wall that forms a second one of the respective walls, a second-shaft proximal portion, a second-shaft distal portion, and a second-shaft lumen extending through the second shaft such that the second-shaft lumen forms a second portion of the interior of the shaft assembly. The second shaft is connected to the first shaft such that the first-shaft distal portion is disposed distal of the second-shaft distal portion and the first-shaft proximal portion. A third of the plurality of tubular components comprises a tip including a tip wall that forms a third one of the respective walls, a tip proximal portion, tip distal portion, and a tip lumen extending through the tip such that the tip lumen forms a third portion of the interior of the shaft assembly. The tip is connected to the first shaft such that the tip distal portion is disposed distal of the first-shaft distal portion and the tip proximal portion.

A choke region is disposed in the shaft-assembly lumen. The choke region has a choke lumen with a choke width that is less than a width of a portion of the shaft-assembly lumen that is located proximal to the choke lumen. The balloon defines an interior volume and is connected to the shaft assembly, such that a proximal portion of the balloon is connected to the second-shaft distal portion and a distal portion of the balloon is connected to the first-shaft distal portion.

Ports are located inside the balloon and disposed through the respective walls, such that the ports define proximal passages between the interior of the shaft assembly and the interior volume of the balloon. A port may be disposed through the first shaft wall, with the first-shaft lumen defining at least a portion of the choke lumen. Alternatively or additionally, a port may also be disposed through the tip wall, with a portion of the tip lumen defining at least a portion of the choke lumen.

A guidewire or another catheter, such as a mapping catheter that provides guidewire functionality, may be disposed through the shaft assembly lumen. The guidewire or other catheter has a width that is equal to or about equal to a width of the choke lumen. When the guidewire is disposed in the choke lumen, irrigation liquid provided from an irrigation pump and into the shaft assembly fills the balloon and pressurizes it into an expanded configuration. When the guidewire is not disposed in the choke lumen, irrigation liquid contained in the balloon can pass back into the shaft assembly lumen via at least one of the ports and then exit the electrophysiology catheter through its tip. As such, the action of withdrawing the electrophysiology catheter into a guide-catheter lumen of a guide catheter causes the guide catheter to compress the balloon and squeeze the liquid contained therein into the shaft assembly and out of the tip of the electrophysiology catheter.

### BRIEF DESCRIPTION OF DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 is a schematic illustration of an invasive medical procedure;
Figure 2 is a top schematic view of a catheter with a balloon in an expanded state;
Figure 3 is a perspective schematic view of a distal portion of the catheter of Figure 2, which also depicts a distal end of a guide catheter and a mapping catheter;
Figure 4 is a side schematic view of a distal portion of the catheter of Figure 2 deployed in the region of a pulmonary vein and its ostium;
Figure 5 is a top schematic view of a distal portion of the catheter of Figure 2;
Figure 6A is a cross-sectional view of the distal portion of the catheter of Figure 2, in which the section plane X-X is indicated in Figure 5;
Figure 6B is a cross-sectional view of the distal portion of the catheter of Figure 2, in which the section plane X-X is indicated in Figure 5;
Figure 6C is a cross-sectional view of the distal portion of the catheter of Figure 2, in which the section plane X-X is indicated in Figure 5;
Figure 7A is a cross-sectional view of the distal portion of the catheter of Figure 2, in which the section plane X-X is indicated in Figure 5;
Figure 7B is a cross-sectional view of the distal portion of the catheter of Figure 2, in which the section plane X-X is indicated in Figure 5;
Figure 7C is a cross sectional view of the distal portion of the catheter of Figure 2, in which the section plane X-X is indicated in Figure 5;
Figure 8 is a cross-sectional view of the distal portion of the catheter of Figure 2, in which the section plane X-X is indicated in Figure 5; and
Figure 8A is a top schematic view of a proximal portion of a catheter;
Figure 9A depicts a tip component of the catheter of Figure 2;
Figure 9B depicts a mid-plane cross section of the tip component of Figure 9A to reflect a seal therein;
Figure 9C depicts the mid-plane cross section of the tip component of Figure 9A with a non-uniform device disposed therethrough;
Figure 9D depicts the mid-plane cross section of the tip component of Figure 9A with the seal depicted in Figure 9B hidden; and
Figure 10 depicts a flow diagram depicting a method of using the catheter of Figure 2.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### System Description

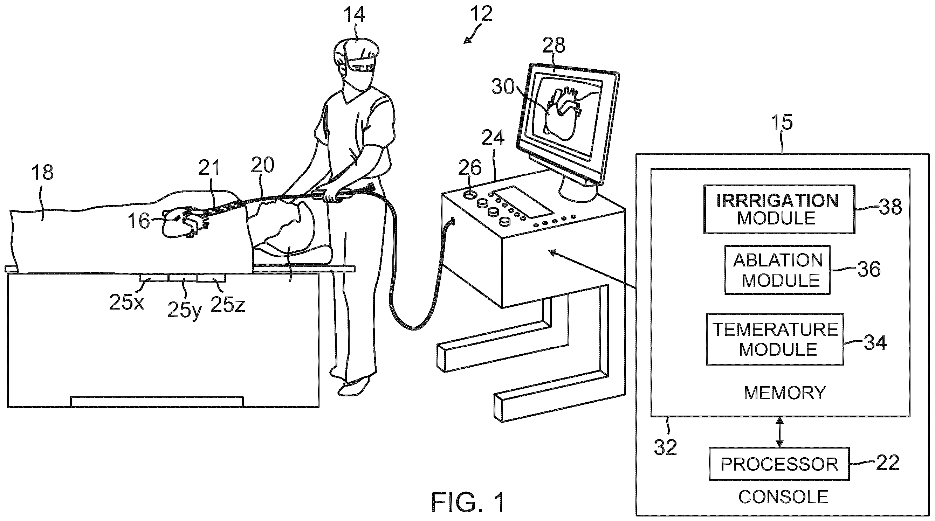

Figure 1 is a schematic illustration of an invasive medical procedure using apparatus 12. The procedure is performed by a medical professional 14, and, by way of example, the procedure in the description hereinbelow is assumed to comprise ablation of a portion of a myocardium 16 of the heart of a human patient 18. However, it is understood that embodiments disclosed herein are not merely applicable to this specific procedure and may include substantially any procedure on biological tissue or on non-biological materials.

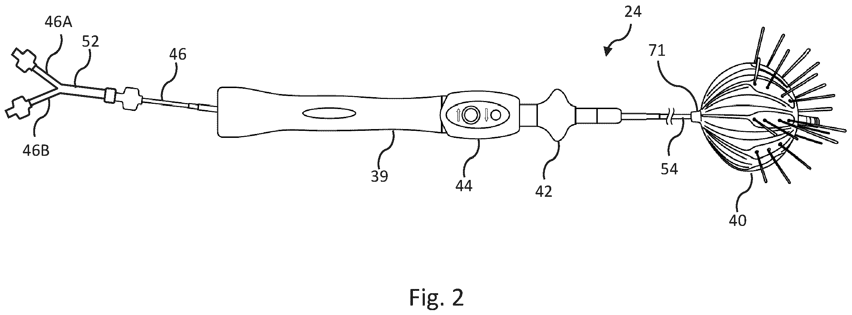

To perform the ablation, medical professional 14 inserts a probe or guide catheter 20 into a sheath 21 that has been pre-positioned in a lumen of the patient. Sheath 21 is positioned so that a distal end of guide catheter 20 enters the heart of the patient. A diagnostic/therapeutic or electrophysiology catheter 24, which is described in more detail below with reference to Figure 2, is deployed through a lumen of the guide catheter 20, and exits from a distal end of the guide catheter 20.

As shown in Figure 1, apparatus 12 is controlled by a system processor 22, which is in an operating console 24 of the apparatus, also shown schematically at reference numeral 15. Console 24 comprises controls 26 and screen 28, which may be used by medical professional 14 to communicate with the processor. As such, screen 28 may comprise a touch screen and controls 26 may comprise, e.g., a mouse or trackball. During the procedure, processor 22 typically tracks a location and an orientation of the distal end of the guide catheter 20, using any method known in the art. For example, processor 22 may use a magnetic tracking method, wherein magnetic transmitters 25X, 25Y and 25Z external to the patient 18 generate signals in coils positioned in the distal end of the guide catheter 20. The CARTO^{®} system (available from Biosense Webster, Inc. of Irvine, California) uses such a tracking method.

The software for the processor 22 may be downloaded to the processor in electronic form, over a network, for example. Alternatively, or additionally, the software may be provided on non-transitory tangible media, such as optical, magnetic, or electronic storage media. The tracking of the distal end of guide catheter 20 may be displayed on a three-dimensional representation 30 of the heart of the patient 18 on screen 28. However, it may be displayed two-dimensionally, e.g., by fluoroscopy or MRI.

To operate apparatus 12, processor 22 communicates with a memory 32, which has many modules used by the processor to operate the apparatus. Thus, the memory 32 comprises a temperature module 34, an ablation module 36, and an irrigation module 38. Irrigation module 38 may be connected to a pump allowing the processor to control the pump, and thus the flow rate of irrigation liquid provided to the catheter. The memory 32 typically comprises other modules, such as a force module for measuring the force on the distal end of guide catheter 20, a tracking module for operating the tracking method used by the processor 22, an electrocardiograph (ECG) module. For simplicity, such other modules are not illustrated in Figure 1. The modules may comprise hardware as well as software elements.

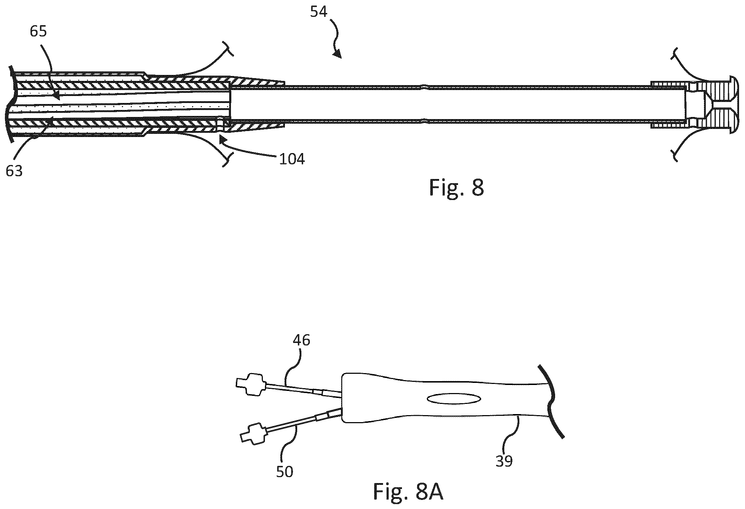

Figure 2 is a schematic perspective view of the electrophysiology catheter 24 in which balloon 40 is in an expanded configuration. Catheter 24 may include a handle 39 comprising, e.g., knob 42 and control 44, which may be used to assist in manipulating and positioning balloon 40. A tubing 46 extends proximally from handle 39 through which irrigation liquid, such as a saline solution, may be pumped. Another catheter, such as a mapping catheter, or a guidewire 48 may also be inserted through electrophysiology catheter 24 via tubing 46, or alternatively, through another tubing 50 (Figure 8A). Where a single tubing 46 extends proximally from handle 39, it may bifurcate into two legs 46A and 46B, and this bifurcation may be provided by Y-connector 52. In this vein, one of leg 46A and 46B may be connected to the irrigation pump while guidewire 48 is disposed through the other leg.

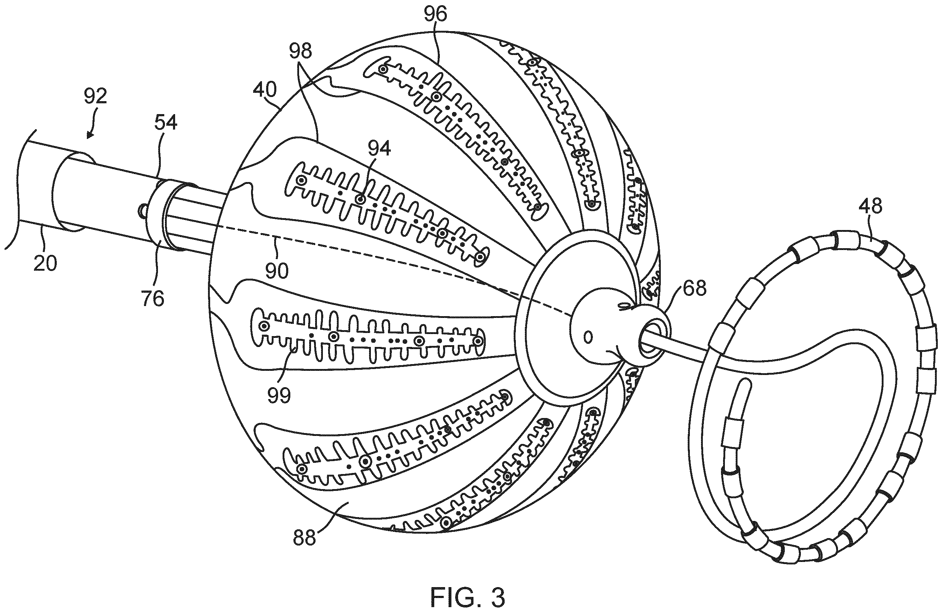

As depicted generally in Figures 3-5, where the electrophysiology catheter 24 is used to ablate an ostium 11 of a lumen, such as a pulmonary vein 13, balloon 40 is supported on a shaft assembly 54. Shaft assembly is tubular, and as such its various components are also tubular. As depicted, shaft assembly 54 comprises at least a first or inner shaft 56 having a first-shaft distal portion 58 and a first-shaft proximal portion 60, and a second or outer shaft 62 having a second-shaft distal portion 64 and a second-shaft proximal portion. Shaft assembly 54 may further comprise a tip 68 having a tip proximal portion 70 and a tip distal portion 72. Shaft assembly 54 may also include a proximal collar 74, a distal collar 76, and a connector 78 having a connector distal portion 84 and a connector proximal portion 86.

First shaft 56 and second shaft 62 are connected directly or indirectly to each other. For example, an indirect connection between first shaft 56 and second shaft 62 may be achieved by directly connecting first-shaft proximal portion 60 to connector distal portion 84 and directly connecting second-shaft distal portion 64 to connector proximal portion 86. These direct connections may be made using at least one of an adhesive and a weld.

Likewise, balloon 40 may be directly or indirectly connected on its proximal end 80 to second-shaft distal portion 64 and on its distal end 82 to first-shaft distal portion 60. For example, an indirect connection may be achieved by connecting balloon proximal end 80 to second-shaft distal portion 64 via connector 78, such as by use of one or more of: an adhesive, a weld, or proximal collar 74 that compresses balloon proximal portion 80 to connector 78. Likewise, balloon distal portion 82 may be connected to first-shaft distal portion 58 via tip 68, such as by use of one or more of: an adhesive, a weld, or distal collar 76 that compresses distal end 82 to tip 68.

The balloon 40 of the electrophysiology catheter 24 has an exterior wall, surface, or membrane 88 of a bio-compatible material, for example, formed from a plastic such as polyethylene terephthalate (PET), polyurethane, or PEBAX^{®}. Shaft assembly 54 defines a longitudinal axis 90 of the balloon 40. The balloon 40 is deployed, in a collapsed configuration, via the lumen 92 of the guide catheter 20, and may be expanded after exiting from the distal end by pressurizing it with the irrigation liquid, as will be detailed below. For a balloon 40 intended to be deployed for use in pulmonary vein ostium 11, the balloon has an ellipsoidal, e.g., spherical shape when expanded. Balloon 40 may have a diameter of between about 25 millimeters and about 35 millimeters, e.g., about 30 millimeters. Membrane 88 of balloon 40 may be formed with irrigation pores or apertures 94 through which fluid (e.g., saline) can exit from the interior of the balloon 40 to outside the balloon for cooling the tissue ablation site at the ostium.

The membrane 88 supports and carries combined electrode and temperature sensing members which are each constructed as a multi-layer flexible circuit electrode assembly 96. The "flex circuit electrode assembly" 96 may have many different geometric configurations. In the illustrated embodiment, the flex circuit electrode assembly 96 has a plurality of radiating substrates or strips 98, upon which are disposed electrodes 99. In the embodiments reflected in the figures, there is one electrode 99 disposed on each of the substrates 98. Thus, for example, where balloon 40 includes ten substrates, it also includes ten electrodes. The substrates 98 are evenly distributed around balloon 40. Each substrate has wider proximal portion that gradually tapers to a narrower distal portion. Further each substrate extends between balloon proximal end 80 and balloon distal end 82.

### Fluid Management

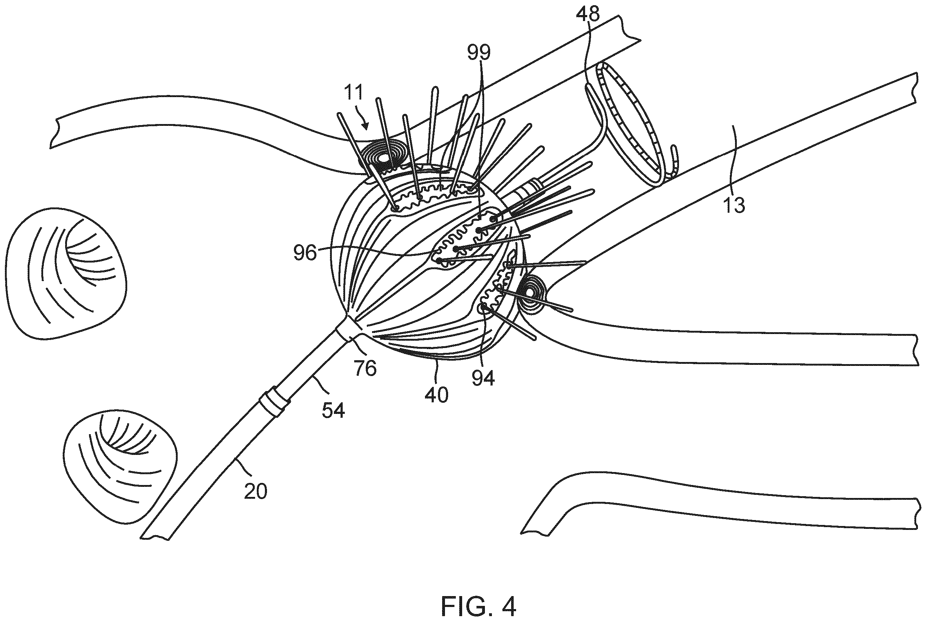

After balloon 40 has been positioned inside of a heart, proximate to or in pulmonary vein ostium 11, the irrigation pump is activated via irrigation module 36 to provide irrigation liquid, e.g., saline through electrophysiology catheter 24, from tubing 46, and into an interior volume 41 of balloon 40. The pump may provide for a first or lesser flow rate and a second or greater flow rate of the irrigation liquid. The lesser flow rate may be between about 0 ml/min and about 10 ml/min, such as between about 2ml/min and about 5 ml/min, e.g., about 3.5 ml/min. The greater flow rate is greater than the lesser flow rate and may be between about 10 ml/min and 50 ml/min, such as between about 25 ml/min and about 35 ml/min, e.g., about 30 ml/min. Initially, the lower flow rate is provided while the balloon remains in a collapsed configuration. Upon increasing the flow rate to the greater flow rate, pressure builds in the interior volume 41 of balloon 40 such that balloon 40 expands to have the spherical shape depicted in the figures. After the balloon has expanded from the collapsed configuration to the expanded and spherical configuration, the irrigation liquid may emerge from apertures 94, either as droplets or as jets as depicted in Figure 4.

Upon completing an ablation at a specific location, balloon 40 is either moved to another cardiac location or removed from heart. Both of these operations typically require lowering the flow rate of the irrigation liquid to the lesser flow rate, collapsing balloon 40, and withdrawing it into guide catheter 20. Yet collapsing balloon 40 and withdrawing it into the guide catheter requires that a sufficient volume of irrigation liquid be expelled from balloon interior volume 41 to avoid damaging balloon 40 or its electrical componentry. Typically, the irrigation liquid is expelled from interior volume 41 by slowly withdrawing balloon 40 into guide catheter 20 such that the tip of guide catheter pushes on membrane 88, thus squeezing the balloon and forcing the irrigation liquid out from interior volume 41 through apertures 94. Applicant has determined that when the lesser flow rate is about 5 ml/min, the expanded balloon has a diameter of about 30 mm, and the balloon's design includes apertures 94 of a sufficient size and number to result in jets of irrigation liquid emerging from apertures 94 when the irrigation liquid is being pumped at the greater flow rate of between about 25 ml/min and about 35 ml/min, it takes about thirty seconds to expel the irrigation liquid from the interior volume by depressing it against the tip of guide catheter 20 while withdrawing it therein, and that attempts to expel the irrigation liquid more quickly by withdrawing balloon 40 into guide catheter 20 more quickly may damage balloon 40 and its electrical componentry. Because balloon 40 may need to be withdrawn into the guide catheter between about five and about fifteen times, e.g., ten times, during a given procedure, Applicant has identified reducing the time it takes to expel the irrigation liquid from internal volume 41 and withdraw balloon 40 into guide catheter as an opportunity for improving the design and usage of electrophysiology catheter 24. Applicant has thus conducted research and development efforts in accordance with this opportunity and presents exemplary solutions below that are embodied in shaft assembly 54.

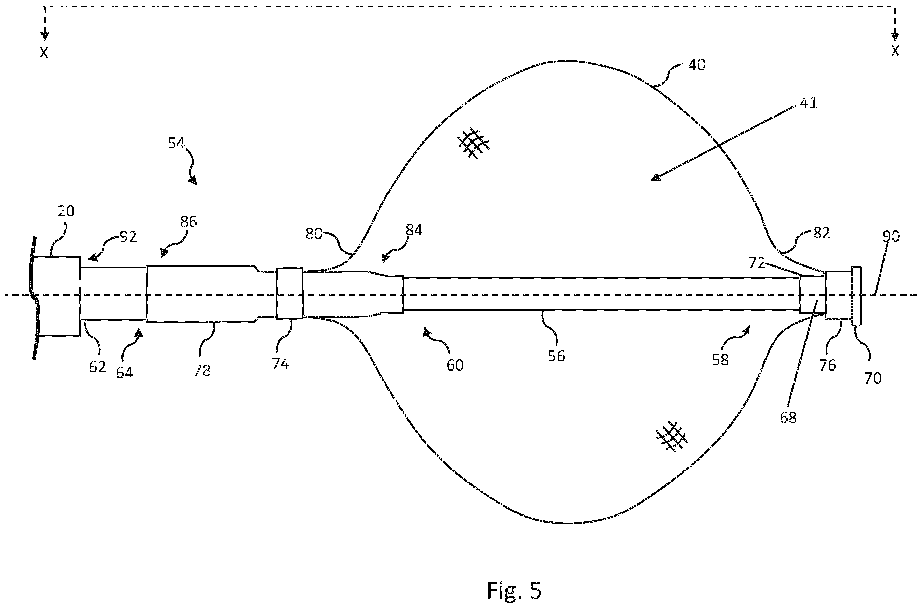

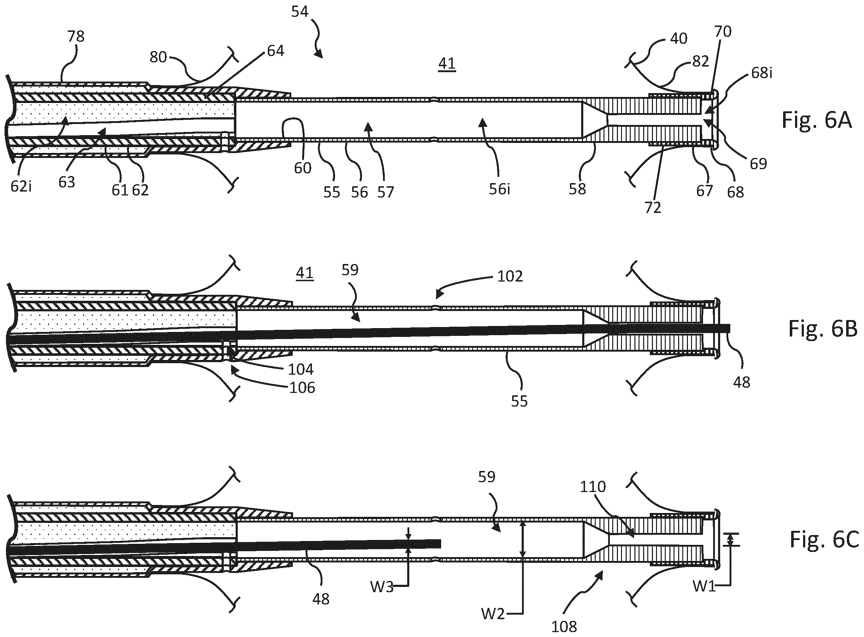

Figures 6A-6C, 7A-7C, and 8 depict a center-plane cross section of shaft assembly 54 as it is depicted in Figure 5, for the purpose of detailing small features and internal features of the shaft assembly. The section plane is parallel to the sheet containing Figure 5, as indicated by section plane X-X, and contains axis 90. In Figures 6A-6C, 7A-7C, and 8, guide catheter 20, collar 74 and collar 76 are hidden, while balloon 40 is depicted in a truncated form such that its distal portion 82 and its proximal portion 80 remain visible. Beginning with Figures 6A-6C, shaft assembly 54 comprises a plurality of tubular components, i.e., first shaft 56, second shaft 62, tip 68, and connector 78, which are connected to each other. Because these components are tubular, each has an exterior wall and a lumen. As shown, first shaft 56 includes a first-shaft wall 55 defining an first-shaft interior 56i comprising a first-shaft lumen 57, second shaft 62 includes a second-shaft wall 61 defining a second-shaft interior 62i comprising a second-shaft lumen 63, and tip 68 includes a tip wall 67 defining a tip interior 68i and a tip lumen 69. Together, the first-shaft interior, the second-shaft interior, and the tip interior, each form a respective portion of an interior of the shaft assembly through which passes shaft-assembly lumen 59. Put another way, first-shaft wall 55, second-shaft wall 61 and tip wall 67 define an interior of shaft assembly 54, while first-shaft lumen 57, second-shaft lumen 63, and tip lumen 69 comprise shaft-assembly lumen 59 in the interior of shaft assembly 54. As seen in Figure 6B, guidewire 48 may pass entirely through shaft-assembly lumen 59. Additionally, first shaft 56 may be disposed entirely inside of balloon 40.

The connection between first shaft 56 and second shaft 62 is made such that first-shaft distal portion 58 is disposed distal of the second-shaft distal portion 64 and the first-shaft proximal portion 60. Connector 78 may be used to facilitate this connection by inserting first-shaft proximal portion 60 inside a distal portion of connector 78 and inserting second-shaft distal portion 64 into a proximal portion of connector 78, and affixing them therein, e.g., with glue or epoxy. In this manner, first shaft 56 and second shaft 62 are indirectly connected to each other via their direct connections to connector 78. Additionally, balloon proximal portion 80 is connected to a distal portion of connector 78, e.g., directly connected by collar 74.

Likewise, the connection between first shaft 56 and tip 68 is made such that tip distal portion 70 is disposed distal of first-shaft distal portion 58 and tip proximal portion 72. First-shaft distal portion 58 is disposed in tip proximal portion 72 and affixed therein, e.g., directly thereto with glue or epoxy. Additionally, balloon distal portion 82 is connected to tip 68, e.g., directly connected by collar 76.

Various ports are also disposed through components of shaft assembly 54. As depicted in Figures 6A-6C, at least one port 102 is disposed through first-shaft wall 55, inside of balloon 40, with two instances being depicted. Thus, port 102 provides a passage between first-shaft lumen 57 and balloon interior 41. Further, at least one port 104 is disposed through second-shaft wall 61 and a connector port 106 is disposed through connector 78 and aligned with port 104 such that ports 104 and 106 are disposed in balloon 40 and together provide a passage between second-shaft lumen 63 balloon interior 41. Thus, ports 102, 104, and 106 provide passages for irrigation liquid to pass between interior volume 41 of balloon 40 and shaft-assembly lumen 59. So oriented, port 102 may be referred to as a distal port and port 104 or ports 104 and 106 may be referred to as a proximal port.

Shaft-assembly lumen 59 also comprises a choke region 108. As depicted in Figures 6A-6C, choke region 108 is located in first-shaft distal portion 58. Choke region 108 has a choke lumen 110 having a choke width W1 that is less than a width W2 of a portion of shaft-assembly lumen 59 that is located proximal to choke region 108. As depicted in Figures 6A-6C, choke lumen 110 thus comprises a portion of first-shaft lumen 57 that has a smaller width than the rest of first-shaft lumen 57. Guidewire 48 should have a width W3 that is equal to or slightly less than width W1 such that guidewire 48 can readily pass through choke lumen 110. W1 may be between about 0.90 mm and about 1.25 mm, e.g., about 1.0 mm. W2 may be between 1.0 mm and about 1.75 mm, e.g., about 1.25 mm, and should be greater than W1. W3 may be between about 0.85 mm and about 0.95 mm, e.g., 0.91 mm, and should be less than or equal to W1.

When guidewire 48 is not disposed in choke lumen 110, as depicted in Figures 6A and 6C, irrigation liquid may flow through shaft-assembly lumen 59 into volume 41 through at least one of ports 102 and 104, and also through choke lumen 110 to exit out of tip 68. Because the irrigation liquid can flow out of tip 68, the irrigation liquid, even when being pumped at the greater flow rate (and also at the lower flow rate) is not capable of maintaining an internal pressure in balloon 40 suitable for an ablation procedure using electrodes 99, or of creating jets of irrigation liquid through apertures 94. Yet, when guide wire 48 is disposed in choke lumen 110, irrigation liquid either cannot pass through choke lumen 110 or is substantially restricted from doing so. Thus, when guide wire 48 is disposed in choke lumen 110, irrigation liquid being pumped at the greater flow rate is capable of maintaining an internal pressure in the balloon suitable for an ablation procedure using electrodes 99, and is also capable of creating jets of irrigation liquid through apertures 94.

This configuration of shaft assembly 54 thus enables shortening the duration of collapsing balloon 40 from its expanded configuration and withdrawing it into guide catheter 20. That is, after balloon 40 has been pressurized into its expanded/spherical configuration by way of providing the irrigation liquid at the greater flow rate while guidewire 48 is disposed in choke lumen 110, the flow rate can be reduced to the lower flow rate and the guidewire 48 can be displaced proximally, e.g., to the position depicted in Figure 6C. From there, withdrawing balloon 40 into guide catheter 20 constricts balloon 40 toward its collapsed configuration, which squeezes the irrigation liquid in internal volume 41 out of port 102 and restricts or prevents any irrigation liquid being pumped at the lower flowrate from entering internal volume 41 through the ports 102 and 104. Instead, the irrigation liquid flows through choke lumen 110 and out of tip 68.

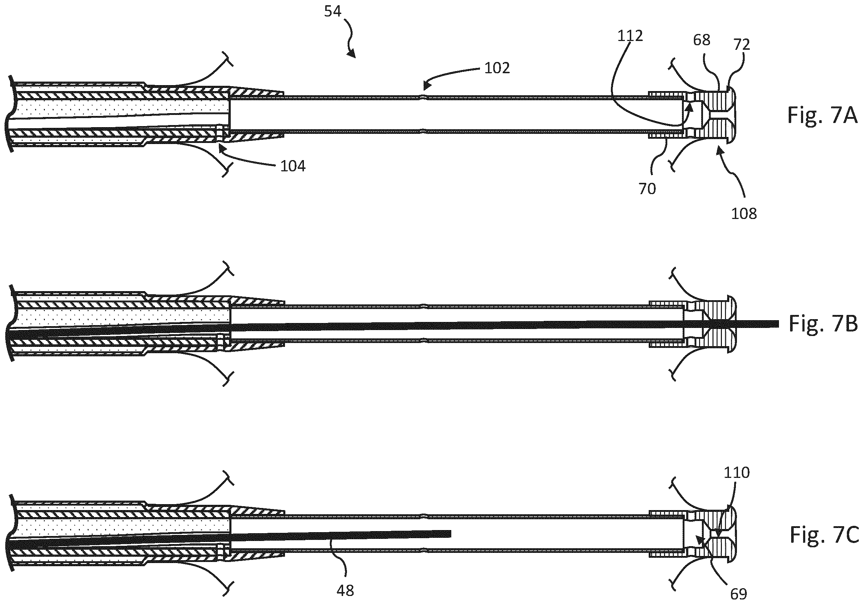

Turning to Figures 7A-7C, another configuration of shaft assembly 54 is depicted. The configuration is similar to the configuration described for Figures 6A-6C, except that: 1) choke region 108 and choke lumen 110 are provided in tip 68; 2) tip 68 includes a tip port 112 positioned proximal to choke region 108 (with two depicted) and in balloon 40. Thus, in this configuration, at least one of ports 102 and 104 serve as the proximal port(s) proximal to choke region 108 and tip port 112, meaning that tip port 112 may also be referred to as the distal port. Likewise, because choke region 108 is provided in tip distal portion 70, choke lumen 110 comprises at least a portion of tip lumen 69. Additionally, tip proximal portion 72 is directly connected to first-shaft distal portion, e.g., with glue or epoxy.

Turning to Figure 8, another configuration of shaft assembly 54 is depicted. This configuration is similar to the configuration described for Figures 6A-6C, except that in Figure 8, in addition to second-shaft lumen 63, a second second-shaft lumen 65 is provided. Second second-shaft lumen 65 provides a passage for guidewire 48, and it is not connected to the irrigation pump for receiving irrigation liquid. Thus, as seen in Figure 8A, two tubes 46 and 50 extend proximally from handle 39. Tube 46 still connects to first second-shaft lumen 63 and remains connectable to the irrigation-fluid pump, while tube 50 connects to second second-shaft lumen 65.

While the foregoing description concerns a design in which the choke width W1 of choke lumen 110 is chosen based on the width of a guidewire 48, which typically has a uniform or nominally uniform cross-section along its length. Yet catheters, notably mapping catheters, may have non-uniform cross sections that have a greater width along at least one longitudinal portion than another longitudinal portion. To facilitate use of a non-uniform device, e.g., a non-uniform catheter or a non-uniform guidewire, that is to be advanced through catheter 24, a seal may be integrated into the tip that is capable of accommodating movement of the non-uniform device and that can maintain a seal against portions of the non-uniform device that have differing widths, even when the non-uniform device is being moved relative to the seal.

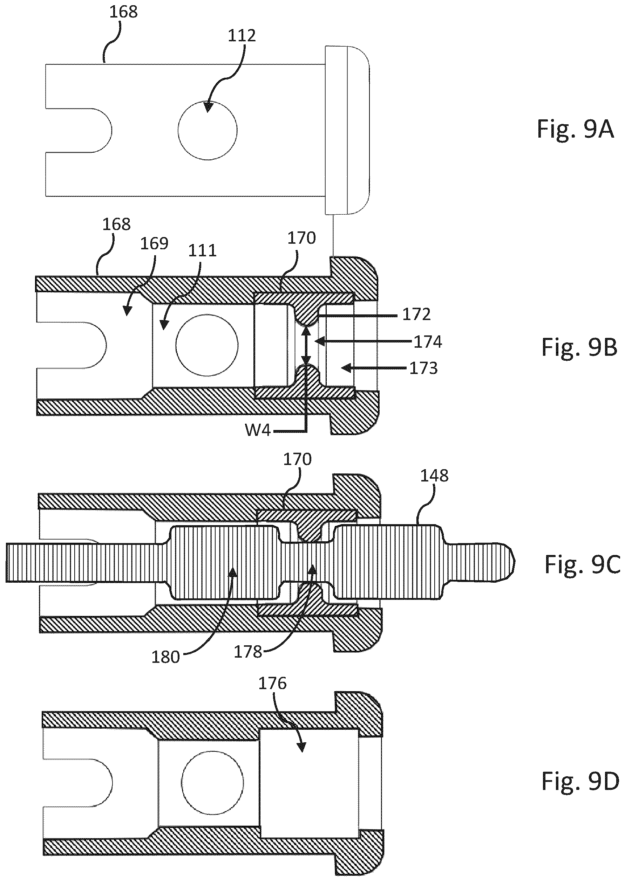

For example, with reference to Figures 9A-9D, tip 168 may include seal 170. Seal 170 may be disposed in choke lumen 111, which as depicted, comprises a portion of tip lumen 169. For example, tip 168 may include a cavity 176 into which seal 170 may be placed and attached to tip 168, e.g., by bonding it therein with an adhesive. As depicted, seal 170 comprises a wiper seal, i.e., a tubular seal having a constriction 172 disposed in a lumen 173 of the seal that defines a bore 174 having a bore width W. The non-uniform device 148, which includes at least one minimal-width portion 178 and one maximal-width portion 180, may be advanced, disposed, and retracted through seal bore 174. At least constriction 172 should be positioned distally to tip port 112, the distal-most port of the catheter, but the entirety of seal 170 may be positioned distally to tip port 112. Of course, if a proximal portion of seal 170 extends proximal to tip port 112, seal 170 should include a port that is aligned with distal tip port 112 so that fluid may pass between interior volume 41 of balloon 24 and shaft-assembly lumen 59.

Contact between seal 170 and non-uniform device 148 may be maintained while the non-uniform device is moved through bore 174 of constriction 172. Thus, a seal between seal 170 and non-uniform device 148 is maintained so long as non-uniform device 148 and constriction 172 contact each other. Yet because the goal of the subject matter presented herein is to enable a user to more rapidly collapse balloon 40, a perfect seal is not necessary so long as fluid-flow through bore 174 is sufficiently restricted while non-uniform device 148 is disposed therein for achieving desired pressures inside of balloon 40 for inflation and production of jets of irrigation fluid. Accordingly, W4, the width of bore 174, may be equal or slightly greater than the minimum width of non-uniform device 148, i.e., the width of minimal-width portion 178 of non-uniform device 148. Appropriate exemplary dimensions include the width of bore 174 being about 0.38 mm to about 0.95 mm, e.g., about .60 mm, for a non-uniform device 148 having a minimum width, i.e., the width of minimal-width portion 178, that ranges between those same dimensions, i.e., between about .38 mm to about 0.95 mm, e.g., about .60 mm. To enable portions of non-uniform device 148 that have a greater width than the minimum width of bore 174 (which can include the entirety of non-uniform device, including minimal-width portion 178) to pass through bore 174 of constriction 172, constriction 172 should be able to flex and bend. Accordingly, seal 170 should be fabricated from a material comprising an elastomer, e.g., a medical-grade silicone or urethane, having a shore A durometer hardness of between about 25 and about 40.

Shaft assembly 54 may include other configurations of ports that enable pressurization of balloon 40 while guidewire 48 is disposed through choke lumen 110 and subsequently enable liquid contained in balloon 40 to readily or rapidly flow out of balloon 40 into shaft-assembly lumen 59, and out of tip 68, particularly in response to compressing balloon 40 against the tip of guide catheter 20 while withdrawing balloon 40 into lumen 92 of guide catheter 20 while guidewire 48 is not disposed in choke lumen 110. For example, any number of ports 102, 104, at 112 may be provided, and in some configurations, only one or two of these ports may be sufficient to accomplish design goals. Conversely, additional ports may need to be provided in certain instances. Any design decision concerning the number and placement of the ports should be determined based on at least the irrigation flow rates used, the diameters of the ports, the size of the balloon, and the speed with which balloon 40 should be collapsed. For example, the diameter of any of ports 102, 104, and 112 may be between about 0.4 mm and 0.8 mm, e.g., 0.6 mm.

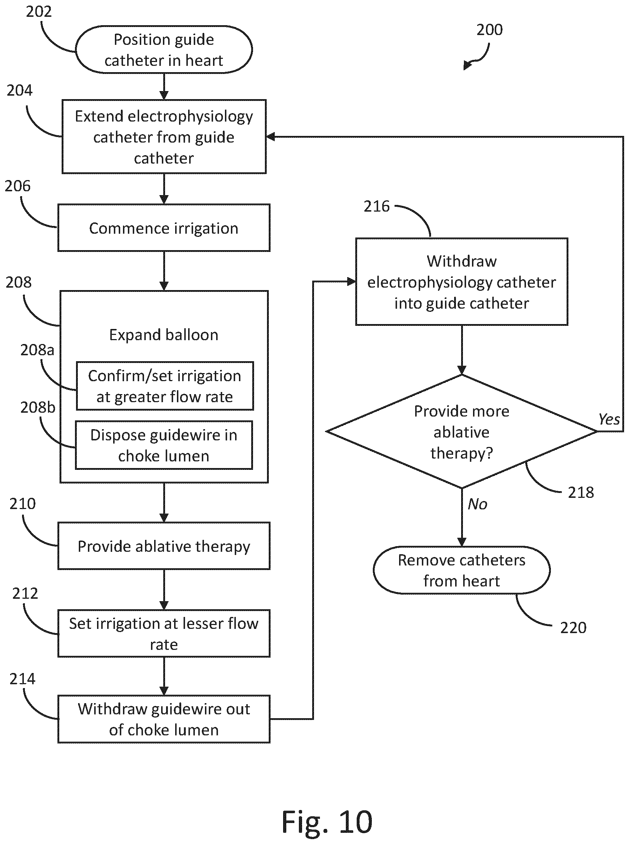

By virtue of the embodiments illustrated and described herein, Applicant has devised a method and variations thereof for using an electrophysiology catheter, e.g., catheter 24, having a balloon disposed on its distal portion, e.g., balloon 40, in which irrigation liquid in the balloon may be quickly expelled from the balloon such that the balloon may be quickly withdrawn into a guide catheter without damaging the balloon or any of the balloon's electrical componentry. The method is depicted as method 200 in the flow chart of Figure 10.

Method 200 commences at step 202, which comprises positioning a distal portion of a guide catheter near to a target location in a human heart. At step 204, the distal portion of the electrophysiology catheter comprising balloon 40 is extended out of the guide catheter, with the balloon in its collapsed configuration. At step 206, irrigation is commenced by activating the irrigation pump with the irrigation module. If the irrigation pump is to be activated before the electrophysiology catheter is extended out of the guide catheter, the pump should be set at the lesser flow rate. After the balloon is disposed outside of the guide catheter, however, the irrigation pump is set at the greater flow rate.

At step 208, the balloon is expanded to its expanded, ellipsoidal configuration. As such, to account for those variations where the pump is activated to pump at the lesser flow rate while the balloon is disposed in the guide catheter, step 208 additionally includes step 208a of confirming that the pump is set to provide the irrigation liquid at the greater flow rate, and if it is not, so setting it. Then, at step 208b, a portion of a guidewire or a non-uniform device, e.g., a mapping catheter, is disposed in the choke lumen. Such blocks or restricts flow of the irrigation liquid out of the tip of the catheter's shaft assembly. Thus, all or most of the irrigation liquid that enters the shaft-assembly lumen must enter the interior volume of the balloon via the shaft assembly's ports. Such pressurizes the balloon into its expanded, ellipsoidal configuration, and results in jets of irrigation liquid emerging from the balloon's apertures.

At step 210, ablative therapy may be provided to cardiac tissue by activating the electrodes with the ablation module. At step 212, the irrigation pump is set to pump the irrigation liquid at the lesser flow rate. At step 214, which begins between about zero seconds and about five seconds after step 212 is completed, the guidewire is withdrawn distally, so that no portion of it remains in the choke lumen.

At step 216, the electrophysiology catheter is withdrawn into the guide catheter so that the tip of the guide catheter compresses the balloon, thus forcing some of the irrigation liquid inside of the balloon to flow back into the shaft-assembly lumen via at least the distal-most port of the shaft assembly, i.e., either port 104 as in Figures 6A-C or port 112 as in Figures 7A-7C. Irrigation liquid could also flow back into the shaft-assembly lumen via any other port, depending on, e.g., the flow rates used and the sizes of the ports. Continued compression of the balloon caused by withdrawing the balloon into the guide catheter thus causes irrigation liquid in the balloon to ultimately flow out of the shaft assembly through the catheter tip. Ultimately, the entire balloon is withdrawn into the guide catheter, at which point the balloon has also returned to its collapsed configuration. For a balloon that has an expanded spherical configuration with a diameter of between about 25 mm and about 35 mm, step 216 may be completed without damaging the balloon or its electrical componentry in less than about fifteen seconds (e.g., between about five seconds and about fifteen seconds) after step 214 is completed. At step 218, a medical professional determines whether to provide additional ablative therapy from the balloon. If so, some or all of the foregoing steps, beginning with step 204, are repeated. If not, at step 220, the method ends by removing the electrophysiology catheter and the guide catheter from the patient's heart.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings. Aspects of the invention:
1. A method of using the electrophysiology catheter of claim 1, the method comprising:
   activating an irrigation pump;
   flowing a liquid from the irrigation pump while a portion of a guidewire is disposed in the choke lumen;
   expanding the balloon with the liquid such that the balloon has an ellipsoidal configuration;
   after the operation of expanding the balloon, displacing the guidewire proximally such that the portion of the guidewire is not disposed is the choke lumen; and
   after the operation of displacing the guidewire proximally, withdrawing the electrophysiology catheter into a guide-catheter lumen of a guide catheter.
2. The method of aspect 1, in which the operation of withdrawing the electrophysiology catheter into the guide-catheter lumen comprises compressing the balloon against a distal end of the guide catheter such that at least some of the liquid in the balloon is forced into the distal port and out of the tip distal portion.
3. The method of aspect 2, in which the operation of flowing the liquid into the interior volume of the balloon includes pumping the liquid at a volume flow rate of between about 10 ml/min and about 50 ml/min.
4. The method of aspect 3, further comprising lessening the flow rate to a lower flow rate before the operation of withdrawing the electrophysiology catheter into the guide-catheter lumen.
5. The method of aspect 4, in which the operation of withdrawing the electrophysiology catheter into the guide-catheter lumen is commenced between about zero seconds and about five seconds after the operation of lessening the volume flow rate.
6. The method of aspect 4, in which the ellipsoidal configuration of the balloon is spherical and has a diameter of between about 25 mm and about 35 mm, and the operation of disposing the balloon entirely in the guide-catheter lumen is completed in less than about fifteen seconds after the operation of lessening the volume flow rate.

## Claims

1. An electrophysiology catheter, comprising:
a shaft assembly comprising:
a plurality of tubular components having respective walls that together define an interior of the shaft assembly that includes a shaft-assembly lumen;
a first of the plurality of tubular components comprising a first shaft including a first-shaft wall that forms a first one of the respective walls, a first-shaft proximal portion, a first-shaft distal portion, and a first-shaft lumen extending through the first shaft such that the first-shaft lumen forms a first portion of the interior of the shaft assembly;
a second of the plurality of tubular components comprising a second shaft including a second-shaft wall that forms a second one of the respective walls, a second-shaft proximal portion, a second-shaft distal portion, and a second-shaft lumen extending through the second shaft such that the second-shaft lumen forms a second portion of the interior of the shaft assembly, the second shaft being connected to the first shaft such that the first-shaft distal portion is disposed distal of the second-shaft distal portion and the first-shaft proximal portion; and
a choke region disposed in the shaft-assembly lumen, the choke region including a choke lumen having a choke width that is less than a width of a portion of the shaft-assembly lumen that is located proximal to the choke lumen;
a balloon defining an interior volume connected to the shaft assembly, such that a proximal portion of the balloon is connected to the second-shaft distal portion and a distal portion of the balloon is connected to the first-shaft distal portion;
a proximal port located inside the balloon and disposed through one of the respective walls, such that the proximal port defines a proximal passage between the interior of the shaft assembly and the interior volume of the balloon; and
a distal port located inside the balloon, and disposed through the one of the respective walls or another of the respective walls, such that the distal port defines a proximal passage between the interior of the shaft assembly and the interior volume of the balloon.

2. The electrophysiology catheter of claim 1, in which the width of the portion of the shaft-assembly lumen that is positioned proximal to the choke lumen is between about 1.0 mm and about 1.75 mm.

3. The electrophysiology catheter of claim 1, in which a third of the plurality of tubular components comprises a tip including a tip wall that forms a third one of the respective walls, a tip proximal portion, tip distal portion, and a tip lumen extending through the tip such that the tip lumen forms a third portion of the interior of the shaft assembly, the tip being connected to the first shaft such that the tip distal portion is disposed distal of the first-shaft distal portion and the tip proximal portion.

4. The electrophysiology catheter of claim 3, in which the tip proximal portion is directly connected to the first-shaft distal portion.

5. The electrophysiology catheter of claim 3, in which the distal port is disposed through the first-shaft wall.

6. The electrophysiology catheter of claim 5, in which the first-shaft lumen defines at least a portion of the choke lumen, and/or in which the distal port is disposed through the tip wall.

7. The electrophysiology catheter of claim 3, in which the tip lumen defines at least a portion of the choke lumen.

8. An electrophysiology catheter, comprising:
a shaft assembly comprising:
a plurality of tubular components having respective walls that together define an interior of the shaft assembly that includes a shaft-assembly lumen;
a first of the plurality of tubular components comprising a first shaft including a first-shaft wall that forms a first one of the respective walls, a first-shaft proximal portion, a first-shaft distal portion, and a first-shaft lumen extending through the first shaft such that the first-shaft lumen forms a first portion of the interior of the shaft assembly;
a second of the plurality of tubular components comprising a second shaft including a second-shaft wall that forms a second one of the respective walls, a second-shaft proximal portion, a second-shaft distal portion, and a second-shaft lumen extending through the second shaft such that the second-shaft lumen forms a second portion of the interior of the shaft assembly, the second shaft being connected to the first shaft such that the first-shaft distal portion is disposed distal of the second-shaft distal portion and the first-shaft proximal portion; and
a choke region disposed in the shaft-assembly lumen, the choke region including a choke lumen having a choke width and a seal disposed in the choke lumen;
a balloon defining an interior volume connected to the shaft assembly, such that a proximal portion of the balloon is connected to the second-shaft distal portion and a distal portion of the balloon is connected to the first-shaft distal portion;
a proximal port located inside the balloon and disposed through one of the respective walls, such that the proximal port defines a proximal passage between the interior of the shaft assembly and the interior volume of the balloon; and
a distal port located inside the balloon, and disposed through the one of the respective walls or another of the respective walls, such that the distal port defines a proximal passage between the interior of the shaft assembly and the interior volume of the balloon.

9. The electrophysiology catheter of claim 8, in which the seal has a tubular form and includes a seal lumen.

10. The electrophysiology catheter of claim 9, in which the width of the portion of the shaft-assembly lumen that is positioned proximal to the choke lumen is between about 1.0 mm and about 1.75 mm.

11. The electrophysiology catheter of claim 9, in which a constriction in the seal lumen defines a bore.

12. The electrophysiology catheter of claim 11, in which the constriction is disposed distal of the distal port, and/or in which a width of the bore is between about 0.38 mm and about 0.95 mm.

13. The electrophysiology catheter of claim 8, in which the seal comprises an elastomer having a shore A durometer hardness between about 25 and about 40.

14. The electrophysiology catheter of claim 8, in which the seal comprises a wiper seal, optionally in which the distal port is disposed through the tip wall, further optionally in which the choke lumen comprises at least a portion of the tip lumen and the seal is disposed in a portion of the choke lumen that comprises at least the portion of the tip lumen.

15. The electrophysiology catheter of claim 8, in which a third of the plurality of tubular components comprises a tip including a tip wall that forms a third one of the respective walls, a tip proximal portion, a tip distal portion, and a tip lumen extending through the tip such that the tip lumen forms a third portion of the interior of the shaft assembly, the tip being connected to the first shaft such that the tip distal portion is disposed distal of the first-shaft distal portion and the tip proximal portion.
